# EUROPEAN PATENT SPECIFICATION

(11) **EP 3 752 071 B1**
(45) Date of publication and mention of the grant of the patent: **20.07.2022**
(21) Application number: 19754830.8
(22) Date of filing: 14.02.2019
(51) Int. Cl.: A61B 8/08, A61B 6/12, A61B 6/00, A61L 29/18, A61B 90/00, A61L 29/08, A61L 29/10

(54) **RADIOPAQUE AND ECHOGENIC COATINGS FOR MEDICAL DEVICES**
RÖNTGENOPAKE UND ECHOGENE BESCHICHTUNGEN FÜR MEDIZINISCHE VORRICHTUNGEN
REVÊTEMENTS RADIO-OPAQUES ET ÉCHOGÈNES POUR DISPOSITIFS MÉDICAUX

(30) Priority: 14.02.2018 US 201862630334 P
(43) Date of publication of application: 23.12.2020
(73) Proprietor: Mitsubishi Chemical Performance Polymers, Inc., Warren, MI 48089 (US)
(72) Inventor: CARROLL, Khristine, North Andover, MA 01845 (US); CHEN, Xiaoxi Kevin, Natick, MA 01760 (US)
(74) Representative: Maiwald Patent- und Rechtsanwaltsgesellschaft mbH
(86) International application number: PCT/US2019/017924
(87) International publication number: WO 2019/161004

(56) References cited:
- EP-A1- 2 561 898
- US-A1- 2011 144 689
- US-A1- 2011 305 881
- US-A1- 2014 148 889
- US-A1- 2016 015 928

## Description

### Cross-Reference to Related Application

This application claims priority of U.S. Provisional Patent Application No. 62/630,334, filed February 14, 2018.

### Field of the Invention

The present invention discloses methods for producing coatings for medical devices that are both echogenic and radiopaque.

### Background of the Invention

Ultrasound has been widely used to guide needle, catheter and guidewire placement and for vascular access, nerve blockade, drainage of pleural or ascitic fluid collections and percutaneous tracheostomy. Ultrasound allows identification of the target and collateral structures and real-time guidance to precisely place needles and other inserted devices.

The visibility of a needle or other inserted devices in ultrasound guided procedures is extremely important. Without accurate identification of the position of the needle it is possible that damage to collateral structures may occur. However, most medical devices have an acoustic impedance similar to that of the tissue into which the device is inserted. Consequently, visibility of the device can be poor and accurate placement can become extremely difficult.

Radiopaque materials, such as bismuth subcarbonate and barium sulfate, have been widely used in the design of various devices such as guidewires or stents that are used during radiological intervention. The radiopacity of a given endovascular device is important since it allows the device to be tracked during the interventional procedure.

EP 2 561 898 A1 discloses a medical device with a radiopaque or echogenic polyurethane coating.

Due to the high density of radiopaque materials, it is possible to use them to provide a contrast for ultrasound visibility. Furthermore, it is advantageous to provide coatings for medical devices that are both echogenic and radiopaque.

### Summary of the Invention

In light of the foregoing, the present disclosure relates to medical device coatings that are both echogenic and radiopaque. More specifically, the coating composition of the present disclosure comprises a polymer matrix dispersed with radiopaque materials. The polymers used in the coating composition preferably adhere strongly to the substrate surfaces and allow a homogeneous dispersion of the radiopaque materials.

The embodiments according to the present invention are directed to a use of a coating for improving ultrasound visibility of a medical device, wherein the coating comprises radiopaque materials dispersed within a polymer matrix, and wherein the radiopaque materials are selected from the group consisting of bismuth subcarbonate, bismuth oxide, bismuth oxychloride and barium sulfate.

### Brief Description of the Figures

FIG. 1 is a drawing representing a substrate coated using echogenic and radiopaque coating comprising a polymer matrix and a dispersion of radiopaque materials.
FIG. 2 shows the comparison of 2 ultrasonograms. The one on the left corresponds to the ultrasonogram of an uncoated stainless-steel needle immersed in water. The one on the right corresponds to the ultrasonogram of a coated stainless-steel needle immersed in water. The coated stainless-steel needle was prepared using the echogenic and radiopaque coating of the subject invention, as described in Example A.

### Detailed Description of the Invention

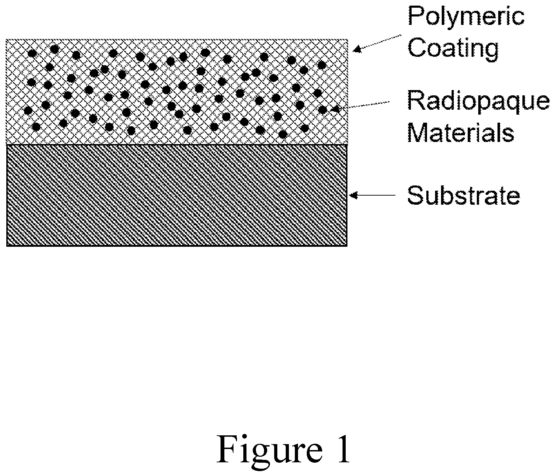

With reference to FIG. 1, the substrate, which is the outer surface of a needle or other medical devices, is coated with a matrix formed by polymer and/or other materials dispersed with radiopaque materials.

The polymers used to form the polymer matrix preferably are biocompatible and have good tensile strength and adhesion to a wide array of metallic and polymeric substrates. Suitable polymers include those that have been used as polymeric coatings for medical devices such as polyurethane (PU), polymethylmethacrylate (PMMA), poly vinylalcohol (PVA), poly-N-vinylpyrrolidone (PVP), polyethylene oxide (PEO), and copolymers thereof. Mixtures and blends of these polymers also can be used. Other matrix based coatings or jackets can also be used.

Radiopaque materials used to be dispersed in the polymer matrix preferably are biocompatible. The radiopaque materials are: bismuth subcarbonate, bismuth oxide, bismuth oxychloride, and barium sulfate.

The radiopaque materials and the polymers can be mixed together in one or more organic solvents to provide a coating composition. Suitable solvents that can be used include, but not limited to, tetrahydrofuran, acetone, methylethylketone, dimethylformamide, dimethyacetamide, ethylene carbonate, propylene carbonate, diglyme, N-methylpyrrolidone, ethyl acetate, ethylene and propylene glycol diacetates, alkyl ethers of ethylene and propylene glycol monoacetates, toluene, xylene and sterically hindered alcohols such as t-butanol and diacetone alcohol. In preferred embodiments, the organic solvent or solvent mixture is evaporative. For example, tetrahydrofuran can be used. The total solid loading can be between about 5 wt.% and about 30 wt.%, where the loading of the radiopaque materials is between about 10 wt.% and about 200 wt.% of that of the polymer.

To improve the echogenicity of a medical device, at least a portion of the surface of the medical device is coated with the present coating composition. Various coating techniques such as spin coating, drop-casting, zone casting, dip coating, blade coating, and spraying can be used, depending on the shape of the medical device. For example, the medical device can be an elongated member such as a catheter, a guidewire, or a needle, or a planar or spherical member such as an implant or a balloon. Typical thickness of the coating can range from about 0.01 mm to about 0.2 mm. The thickness achieved by one application of the coating composition will depend on the viscosity of the coating composition, the coating method, as well as the speed and the temperature at which the coating is applied. In some embodiments, multiple applications of the coating may be needed to build up the required thickness. The coating is then allowed to dry.

### EXAMPLES

### Example A

Stainless-steel needles were coated. A coating solution was prepared by first dissolving 5% (w/v) ChronoFlex AL in tetrahydrofuron, followed by mixing 10% (w/v) bismuth subcarbonate in the ChronoFlex solution until a homogeneous solution is obtained. Stainless-steel needles were then dipped into the coating solution and lifted up slowly. The stainless-steel needles were then dried at room temperature for 30 minutes.

### Example B

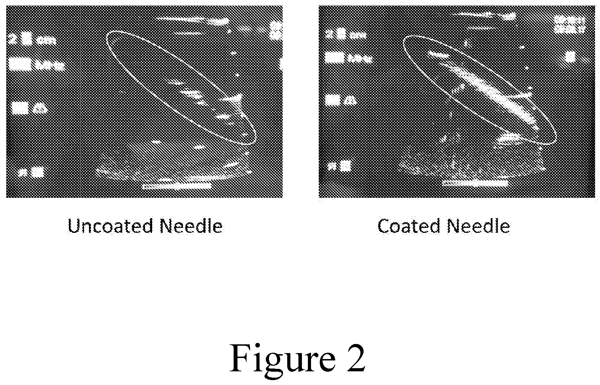

Stainless-steel needles prepared using the durable echogenic coating of the subject invention as described in Example A were compared with uncoated stainless-steel needles for ultrasound visibility in water. FIG. 2 shows the comparison of 2 ultrasonograms. The one on the left corresponds to the ultrasonogram of an uncoated stainless-steel needle immersed in water. The one on the right corresponds to the ultrasonogram of a coated stainless-steel needle immersed in water. The coated needle has significantly improved ultrasound visibility compared to the uncoated needle.

## Claims

1. Use of a coating for improving ultrasound visibility of a medical device, wherein the coating comprises radiopaque materials dispersed within a polymer matrix, and wherein the radiopaque materials are selected from the group consisting of bismuth subcarbonate, bismuth oxide, bismuth oxychloride and barium sulfate.

2. The use of Claim 1, wherein the polymer matrix includes polyurethanes.

3. The use of Claim 1, wherein the polymer matrix includes polycarbonate-based urethanes.

4. The use of Claim 1, wherein the polymer includes silicones.

5. The use of Claim 1, wherein the coating is prepared by dipping the substrate in the coating solution containing the matrix and the radiopaque materials.

6. The use of Claim 5, wherein the coating solution contains 0.1-20% (weight to volume) of the combined matrix and radiopaque materials.

7. The use of Claim 5, wherein the solvent is either tetrahydrofuran, dimethylacetamide, or a mixture of both.

8. The use of Claim 5, wherein a multiple dipping process is used to obtain the coating.

## Patentansprüche

1. Verwendung einer Beschichtung zur Verbesserung der Ultraschallsichtbarkeit einer medizinischen Vorrichtung, wobei die Beschichtung röntgendichte Materialien umfasst, die in einer Polymermatrix dispergiert sind, und wobei die röntgendichten Materialien ausgewählt sind aus der Gruppe, bestehend aus Bismutsubcarbonat, Bismutoxid, Bismutoxychlorid und Bariumsulfat.

2. Verwendung gemäß Anspruch 1, wobei die Polymermatrix Polyurethane enthält.

3. Verwendung gemäß Anspruch 1, wobei die Polymermatrix Urethane auf Polycarbonatbasis enthält.

4. Verwendung gemäß Anspruch 1, wobei das Polymer Silikone enthält.

5. Verwendung gemäß Anspruch 1, wobei die Beschichtung durch Eintauchen des Substrats in die Beschichtungslösung, die die Matrix und die röntgendichten Materialien enthält, hergestellt wird.

6. Verwendung gemäß Anspruch 5, wobei die Beschichtungslösung 0,1-20 % (Gewicht/Volumen) der kombinierten Matrix und der röntgendichten Materialien enthält.

7. Verwendung gemäß Anspruch 5, wobei das Lösungsmittel entweder Tetrahydrofuran, Dimethylacetamid oder eine Mischung aus beiden ist.

8. Verwendung gemäß Anspruch 5, wobei die Beschichtung durch mehrfaches Eintauchen erhalten wird.

## Revendications

1. Utilisation d'un revêtement pour améliorer la visibilité aux ultrasons d'un dispositif médical, dans laquelle le revêtement comprend des matériaux radio-opaques à l'intérieur d'une matrice polymère, et dans laquelle les matériaux radio-opaques sont choisis dans le groupe constitué par le sous-carbonate de bismuth, l'oxyde de bismuth, l'oxychlorure de bismuth et le sulfate de baryum.

2. Utilisation selon la revendication 1, dans laquelle la matrice polymère englobe des polyuréthanes.

3. Utilisation selon la revendication 1, dans laquelle la matrice polymère englobe des uréthanes à base de polycarbonate.

4. Utilisation selon la revendication 1, dans laquelle le polymère englobe des silicones.

5. Utilisation selon la revendication 1, dans laquelle le revêtement est préparé par immersion du substrat dans la solution de revêtement contenant la matrice et les matériaux radio-opaques.

6. Utilisation selon la revendication 5, dans laquelle la solution de revêtement contient 0,1 à 20 % (en poids/volume) de la matrice et des matériaux radio-opaques combinés.

7. Utilisation selon la revendication 5, dans laquelle le solvant est soit le tétrahydrofurane, soit le diméthylacétamide, soit un mélange des deux.

8. Utilisation selon la revendication 5, dans laquelle un traitement par immersions multiples est utilisé pour que le revêtement soit obtenu.
